(19) **Europäisches Patentamt / European Patent Office / Office européen des brevets**

(11) **EP 3 323 350 A2**

(12) **EUROPEAN PATENT APPLICATION**

(43) Date of publication:
**23.05.2018 Bulletin 2018/21**

(51) Int Cl.:
**A61B 8/08** (2006.01)     **A61B 8/06** (2006.01)
**A61B 8/02** (2006.01)

(21) Application number: **17201383.1**

(22) Date of filing: **13.11.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **16.11.2016 US 201662423189 P**

(71) Applicant: **Huntleigh Technology Limited Dunstable, Bedfordshire LU5 5XF (GB)**

(72) Inventor: **Gough, Nigel AJ Pontyclun, CF72 8JT (GB)**

(74) Representative: **Zacco GmbH Bayerstrasse 83 80335 München (DE)**

(54) **ULTRASOUND DOPPLER MONITORING SYSTEM**

(57)    The disclosure is directed to a novel Doppler ultrasound system (10) and method for monitoring physiological parameters, such as blood flow and heart rate in which signal clarity and accuracy is improved by actively filtering out system, environmental and background signal noise.

FIG. 1

## Description

## BACKGROUND

### 1. Field of the Art

The present disclosure relates to a Doppler ultrasound system and method of filtering noise when monitoring blood flow or heart rate, in particular arterial blood flow, venous blood flow and/or the heart rate of a fetus. Ultrasound is used to monitor blood flow and/or heart rate by means of a transducer which is placed in contact with a patient and transmits high frequency sound waves. Reflected echoes and signals received by the transducer are processed to remove undesirable noise to clearly discern and accurately detect and analyze physiological aspects relating to the patient's blood flow and fetal heartbeat.

### 2. Description of the Related Technology

Obstetric and vascular Doppler ultrasound may be used to assess fetal heartbeat and blood flow. For example, conventional Doppler ultrasound fetal heart rate systems insonate the fetal heart and surrounding tissue with high frequency sound. Echoes from internal tissues undergo Doppler shift proportional to the relative velocity of reflecting surface and transducer. The received ultrasound signals are demodulated to convert the Doppler signal to the audible range and further autocorrelated in a time domain for data analysis to extract and/or identify a fetal heart rate.

Arterial and venous blood flow may be similarly assessed using vascular Doppler ultrasound. Ultrasound waves reflected from a patient's blood vessel and surrounding tissue are received by the transducer, digitized and processed for audio and visual waveform presentation. The resultant signals are relied upon to assess the condition of blood flow, identify stenosis and abnormalities and detect the presence of clots and aneurysms.

Signal clarity and accuracy, however, can be compromised by background, environmental and system noise. In particular, handheld Doppler systems suffer from noise caused by the thermal effect of system electron flow and collisions, which is typically magnified by high gain amplifiers in the Doppler probe. Typically such wide band, thermal noise is manifested as a continuous background hissing sound or white noise.

Additionally, ultrasound transmission gel placed on the end of a transducer may be another source of noise. High power, predominately low bandwidth noise can result when the gel contacts the transducer and/or is moved, together with the transducer, about a patient's skin. Change in impedance of the gel due to presence of air bubbles creating reflections, gel movement and the inherent difference in the impedance between the gel and transducer tip all create Doppler shifts resulting in loud, crackling sounds.

Some Doppler systems allow a choice of ultrasound frequency to target and improve signal accuracy. This is useful because attenuation of ultrasound in tissue is proportional to frequency, the sound from a lower frequency transducer penetrating to a greater depth than a higher frequency. Therefore, the user will select a low frequency when they require greatest range but will select a higher frequency to avoid picking up unwanted echoes from deep organs or tissue. These techniques, however, fail to redress inherent system noise or environmental noise that is within the selected frequency. Furthermore, an incorrect or an insufficiently broad frequency selection may result in loss of critical data.

Certain obstetric systems use pulsed Doppler ultrasound to improve signal-to-noise ratio (SNR) by gating the ultrasound receiver such that it only accepts signals within a certain range of times after the ultrasound pulse is transmitted. Such techniques also fail to filter out inherent system and environmental noise, such as thermal and gel noise. These signal processing methods merely limit the opening and closing times of the gates to correspond to a desired transit time for the ultrasound and thus determine a maximum and minimum operating range for the ultrasound beam.

The Doppler ultrasound system of the present application includes a novel digital signal processor and noise filter that improves SNR and signal accuracy by addressing the problem of inherent system, background and/or environmental noise.

## SUMMARY

In accordance with a first exemplary embodiment of the application, a Doppler ultrasound monitoring system includes a digital signal processor for processing Doppler ultrasound signals reflected from a patient, wherein the signal processor: applies a fast Fourier transform to a IQ signal derived from the reflected Doppler ultrasound signals, calculates a maximum frequency envelope adapted for filtering out inherent thermal system noise and applies the maximum frequency envelope to the IQ signal to filter thermal noise. The system further includes a visual and/or audio system for generating a visual and/or audio signal derived from the thermal noise filtered IQ signal.

In accordance with a second exemplary embodiment of the application, a Doppler ultrasound monitoring system includes a digital signal processor for processing Doppler ultrasound signals reflected from a patient and an visual and/or audio system for generating an visual or audio signal from a fast Fourier transform processed IQ signal derived from the reflected Doppler ultrasound signals. The signal processor applies a fast Fourier transform to an IQ signal derived from the reflected Doppler ultrasound signals. The signal processor suppresses an amplitude of the generated audio signal for a predetermined period of time upon the occurrence of at least one of: a determination that the fast Fourier transform processed IQ signal is substantially symmetrical, a dynamically assessed IQ signal to gel noise ratio is less than 1; and/or an average of a first set and last set of values of a magnitude frequency spectrum derived from the fast Fourier transform processed IQ signal exceeds a predetermined threshold indicative of gel noise.

In one embodiment, the volume of the suppressed audio signal is linearly increased until full amplitude is achieved in 0.5 seconds.

In accordance with a third exemplary embodiment of the application, a method for filtering thermal noise in a handheld Doppler ultrasound system includes the steps of: receiving Doppler ultrasound signals reflected from a patient with a transducer, using a digital signal processor to apply a fast Fourier transform to a IQ signal derived from the reflected Doppler ultrasound signals, calculating a maximum frequency envelope adapted for filtering out inherent thermal system noise, applying the maximum frequency envelope to the IQ signal to filter out thermal noise and generating an audio or visual signal derived from the thermal noise filtered IQ signal.

In accordance with a fourth exemplary embodiment of the application, a method for filtering gel noise in a handheld Doppler ultrasound system includes the steps of: receiving Doppler ultrasound signals reflected from a patient with a transducer, using a digital signal processor to apply a fast Fourier transform to a IQ signal derived from the reflected Doppler ultrasound signals, and suppressing an amplitude of an audio and/or visual signal generated from the fast Fourier transform processed IQ signal for a predetermined period of time upon the occurrence of at least one of: a determination that the fast Fourier transform processed IQ signal is substantially symmetrical, a dynamically assessed IQ signal to gel noise ratio is less than 1; and/or an average of a first set and last set of values of a magnitude frequency spectrum derived from the fast Fourier transform processed IQ signal exceeds a predetermined threshold indicative of gel noise.

## BRIEF DESCRIPTION OF THE DRAWINGS

FIG. 1 shows a diagram of an exemplary handheld ultrasound Doppler system that may be used for vascular and/or obstetric applications;

FIG. 2 shows a flow chart illustrating exemplary signal processing for one embodiment of a vascular monitoring system, wherein the block diagram shows front end signal processing;

FIG. 3 shows a flow chart illustrating pre FFT signal conditioning, in which the I and Q packet of FIG. 2 is formatted and interleaved to form a IQ complex;

FIG. 4A shows a flow chart illustrating one embodiment of complex FFT pathways, in which the IQ complex of FIG. 3 is further processed by a CFFT to generate visual and audio data;

FIG. 4B shows a flow chart illustrating another embodiment of complex FFT pathways, in which the IQ complex of FIG. 3 is further processed by a CFFT to generate visual and audio data;

FIG. 5 is graph of a time domain signal from the I input channel showing exemplary representations of thermal signals, gel signals and physiological vascular signals;

FIG. 6 is a thermal noise frequency spectrum from an ultrasound probe in which the transmission gel is applied to the transducer and the thermal noise threshold is shown;

FIG. 7 is the thermal noise frequency spectrum of FIG. 6 in which the thermal noise filter has been applied to the spectrum and the thermal noise has been zeroed out before audio reconstruction;

FIG. 8 is a gel noise frequency spectrum showing from an ultrasound probe in which the transmission gel is applied to the transducer and both a thermal noise threshold and gel noise threshold is shown;

FIG. 9A is a gel noise frequency spectrum of FIG. 8, in which an exemplary gel noise filter has been applied to the spectrum;

FIG. 9B is a gel noise frequency spectrum of FIG. 8, in which another exemplary gel noise filter has been applied to the spectrum;

FIG. 10 is a blood flow frequency spectrum showing a Doppler blood flow signal and both a thermal noise and gel noise threshold;

FIG. 11A is the blood flow frequency spectrum of FIG. 10, in which one embodiment of thermal noise and gel noise filters have been applied to the spectrum;

FIG. 11B is the blood flow frequency spectrum of FIG. 10, in which another embodiment of thermal noise and gel noise filters have been applied to the spectrum;

FIG. 12 shows a flow chart illustrating exemplary signal processing for one embodiment of a fetal heart monitoring system, wherein the block diagram shows front end signal processing;

FIG. 13 shows a flow chart illustrating one embodiment of complex FFT pathways, in which the IQ complex of FIG. 12 is further processed by FFT real to generate visual and audio data; and

FIG. 14 shows the details of an autocorrelation function of the flow chart of FIG. 12 with ACF and FHR determinations.

## DETAILED DESCRIPTION OF THE EXEMPLARY EMBODIMENTS

For illustrative purposes, the principles of the present disclosure are described by referencing various exemplary embodiments. Although certain embodiments of the invention are specifically described herein, one of ordinary skill in the art will readily recognize that the same principles are equally applicable to, and can be employed in other systems and methods. Before explaining the disclosed embodiments of the present disclosure in detail, it is to be understood that the disclosure is not limited in its application to the details of any particular embodiment shown. Additionally, the terminology used herein is for the purpose of description and not of limitation. Furthermore, although certain methods are described with reference to steps that are presented herein in a certain order, in many instances, these steps may be performed in any order as may be appreciated by one skilled in the art; the novel method is therefore not limited to the particular arrangement of steps disclosed herein.

It must be noted that as used herein and in the appended claims, the singular forms "a", "an", and "the" include plural references unless the context clearly dictates otherwise. As well, the terms "a" (or "an"), "one or more" and "at least one" can be used interchangeably herein. It is also to be noted that the terms "comprising", "including", "composed of' and "having" can be used interchangeably.

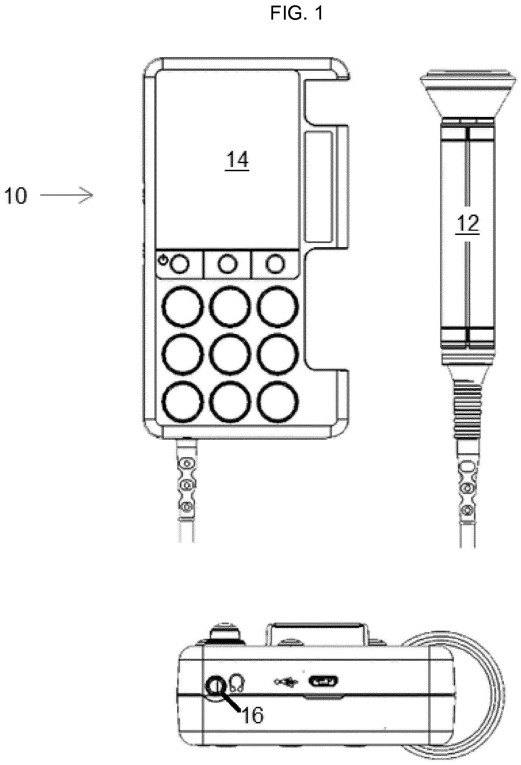

The present disclosure is directed to a novel Doppler ultrasound system and method for monitoring physiological parameters, such as blood flow and heart rate. Referring to the embodiments of FIGS 1-11B, exemplary ultrasound Dopplers 10 and their digital signal processing systems 20 are illustrated. As shown in FIG 1, Doppler 10 may be configured as a handheld, continuous monitoring, vascular ultrasound Doppler including a transducer or probe 12, a display 14 and speakers or audio connection 16 and microcontroller 18 (not shown). A range of different probes 12, such as but not limited to venous photoplethysmography (VPPG) probes for vein examination and pulsed Doppler 8 MHz intraoperative probes, may interchangeably form part of, may be operatively associated with and/or may be connected to a controller or other component of Doppler 10. In one embodiment, Doppler 10 may have the ability to identify and indicate via display 14 blood flow direction and magnitude relative to transducers 12 and/or generate an audio output delivered to an audio system 16 either as a mono signal to an integrated loud speaker or stereo signal separation for forward and reverse flow to headphones. A suitable microcontroller 18 capable of supporting signal processing functions of Doppler 10 may be selected, such as a microcontroller with a digital signal processing (DSP) numerical core. In one embodiment, microcontroller 18 may be capable of sampling the I and Q signals simultaneously at a sampling frequency of up to 40 kHz. In another embodiment microcontroller 18 may have two digital to analogue converters for the forward and reverse separated audio signals. An exemplary microcontroller may be the Freescale MK22FX512VLQ12 with ARM Cortex-M4 DSP core.

In use, transducer 12 of Doppler 10 is placed against a patient's skin, e.g. a patient's arms, legs, foot, abdomen, etc., and ultrasound waves are directed to a target physiological region. Echoes reflected from the target region and the surrounding patient tissue are received by the transducer, digitized and processed for audio and visual waveform output.

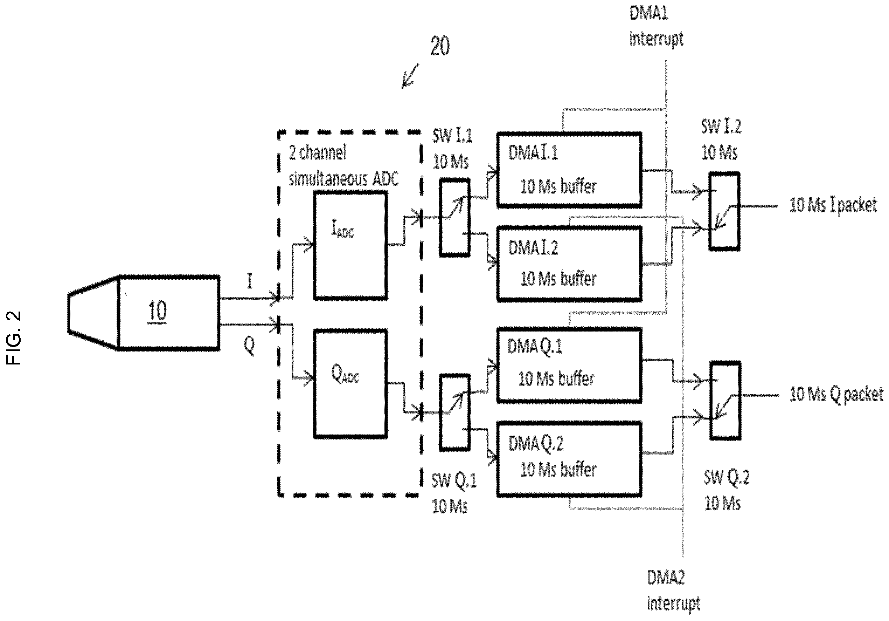

Referring now to FIG. 2, exemplary digital signal processing applied to the received Doppler shifted carrier frequency at the probe face is treated by analogue filtering within the probe to produce the phase quadrature I and Q audio band signals at the probe output. The phase relationship between the incoming I and Q signals is preserved to prevent cross talk between channels throughout the DSP processing system, thus ensuring that errors in measurement of forward and reverse flow Doppler signal energy and associated parameters are minimal. I and Q signals are therefore sampled simultaneously.

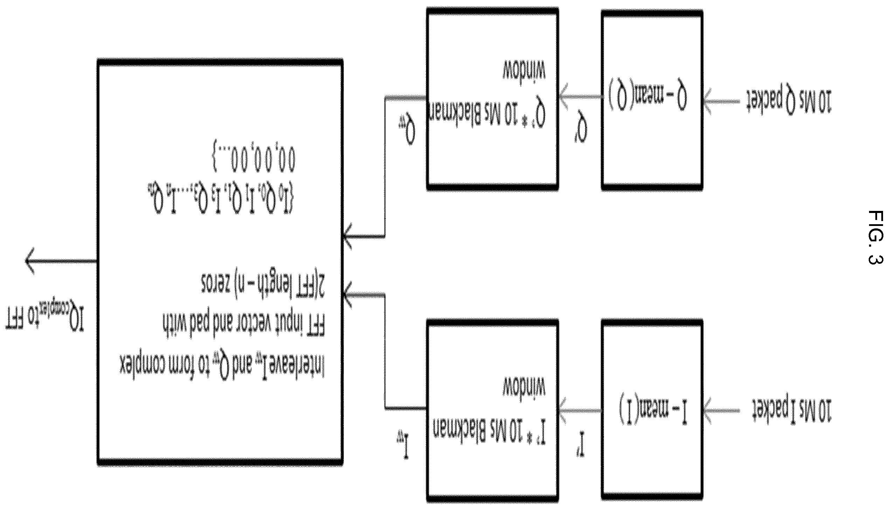

FIG. 2 shows I and Q signals from the Doppler probe entering a two channel simultaneous analog to digital converter (ADC). The sampling rate of the ADC is determined by the potential maximum frequency of the I and Q Doppler signals. For each channel, the output of the ADC is fed via switches SWI.1 and SWQ.1 into one of two DMA controlled 10 ms buffers, DMAI.1 and DMAI.2 for the I channel and DMAQ.1 and DMAQ.2 for the Q channel. When the paired I and Q 10 ms buffers are full, SWI.1 and SWQ.1 are switched so that the other paired 10 ms buffers can receive samples from the ADC. At the same time the data in the 10 ms buffers that have just been filled is channeled by SWI.2 and SWQ.2 as 10 ms I and Q packets to the 40 ms first in first out (FIFO) buffers in FIG 3.

Referring to FIG. 3, the I and Q data packets are then conditioned and formatted to facilitate interleaving of the I and Q data to prepare it for complex fast Fourier transform (CFFT) processing. Before the new 10 ms packets are placed in the 40 ms FIFOs, the most recent 30 ms of data are shifted down the FIFOs by about 25% causing the oldest 10 ms packet to be overwritten. The most recent 10 ms packet is then placed in the top 25% of the FIFOs. The data in these 40 ms FIFOs is referred to as I' and Q'. The data is then conditioned for the CFFT which is achieved by fist subtracting the mean level from I' and Q' and then multiplying by a 40 ms tapering window, W, according to the follow formulas:

$$I_w = (I' - \text{mean}(\ I'\ )) * W;$$

and

$$Q_w = (Q' - \text{mean}(\ Q'\ )) * W.$$

To form the complex input signal for the CFFT $I_w$ and $Q_w$ is interleaved and zero padded to create an input vector twice the length of the FFT. For example, a sampling frequency, $f_s$, 0f 25000 samples per second will yield 250 samples in 10 ms. Therefore, the 40 ms long $I_w$ and $Q_w$ will each have 1000 samples yielding an interleaved input vector of 2000 samples (each pair representing a complex number), $IQ_{complex}$. The nearest power of two fast Fourier transform (FFT) will be 1024 points long. Therefore, $I_w$ and $Q_w$ will have to be padded with an extra 24 zeros each. Or, $IQ_{complex}$ can be padded with 48 zeros. Normally, zero padding need only be applied to one end of the FFT input signal with no effect upon the frequency spectrum. However, after relevant processing, it is intended to use the inverse FFT to recover audio signals. So as to preserve the fidelity of the audio output, it is desirable to pad $IQ_{complex}$ symmetrically. That is, put half the number of zeros at the beginning of the 40 ms complex window and the other half at the end.

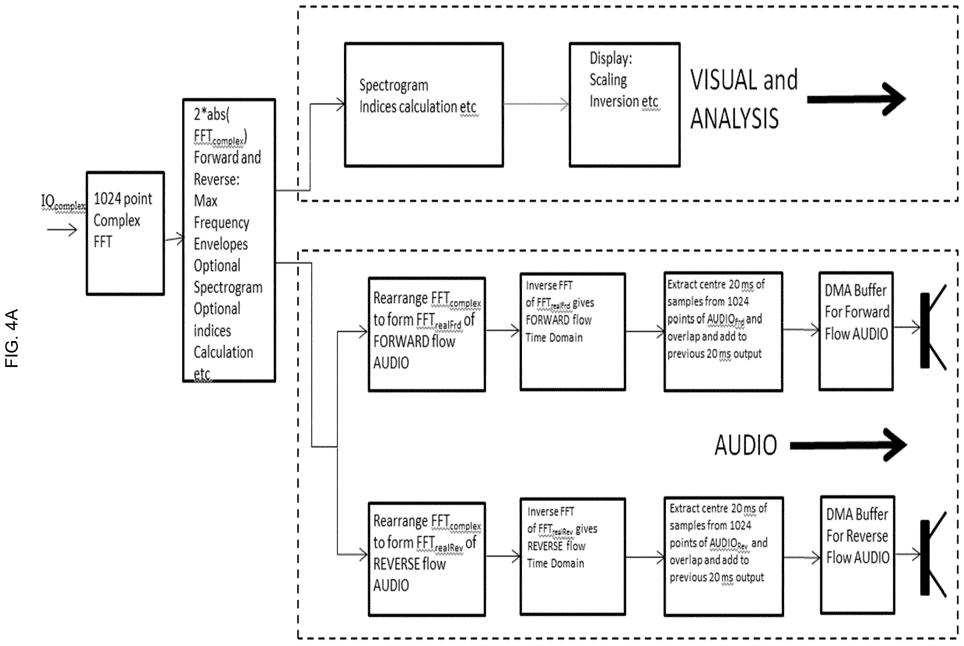

As illustrated in FIG 4A, the resultant interleaved $IQ_{complex}$ is then passed through a complex FFT. The output of the FFT is a complex spectrum, $FFT_{complex}$. The first half of the spectrum represents positive frequencies in the range 0 to $f_s/2$ whereas the second half represents frequencies from 0 to $-f_s/2$. The negative frequencies are related to blood velocities travelling toward the Doppler probe and the positive frequencies to velocities away from the Doppler probe. The $FFT_{complex}$ signal (e.g. 2* abs ($FFT_{complex}$)) may be used to calculate and determine a maximum frequency envelope and filter parameters which may be applied to the $FFT_{complex}$ signal to establish a thermal noise filter and/or gel noise filter. For example, the magnitude of the $FFT_{complex}$ signal may be used, or in another embodiment, 2* abs ($FFT_{complex}$) may also be used. For purposes of the present disclosure, gel noise may be inclusive of transducer movement and noise in general and may be interchangeably referenced as transducer noise.

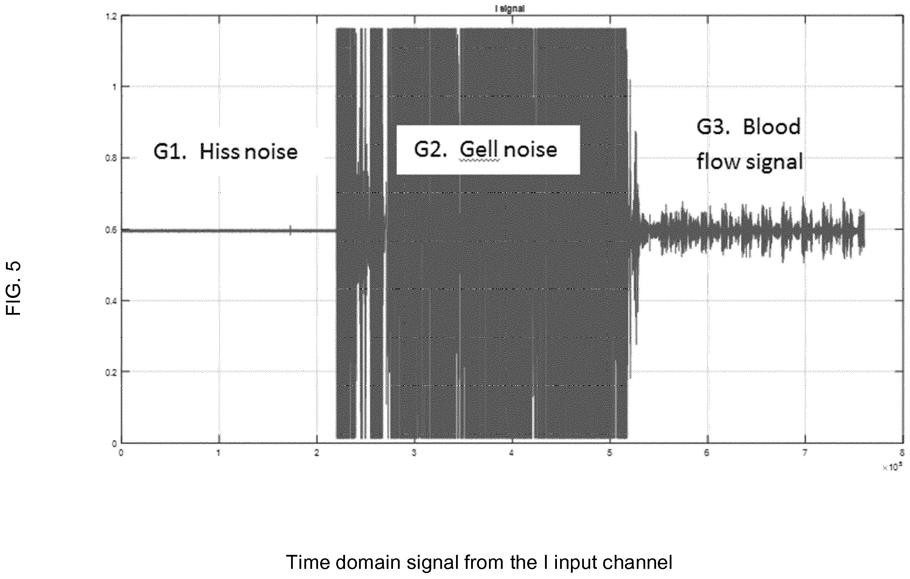

FIG. 5 shows an exemplary time domain signal from the I input channel illustrating a few types of noise that may be present in a ultrasound Doppler system, including thermal noise G1, gel noise G2 and blood flow signal artefacts G3. The gel noise G2 is representative of a sample noise signal when gel is applied to the transducer 12 and during movement of the transducer and gel on a patient's skin when seeking a target blood vessel for examination. Blood flow signal G3 is representative of a sample signal typical of a radial artery. Although the G1 thermal noise may appear small in comparison to the gel noise G2 and the blood flow G3 signal, the thermal noise may still interfere with the detection of low level blood flow signals.

To mitigate and suppress any unwanted noise, in one embodiment, the maximum frequency envelope of the magnitude of the $FFT_{complex}$ signal is used to determine noise filter parameters. As illustrated, the $FFT_{complex}$ signal is conditioned to determine a magnitude of the $FFT_{complex}$ signal (e.g. 2* abs ($FFT_{complex}$)) for forward and reverse flow. Each point in this maximum frequency spectrum is calculated every 10 ms. The maximum frequency in the spectrum is set at a power point. In one embodiment, the power point may be correlated to a confidence level in identification of a target signal and/or signal strength of a target signal, e.g. blood flow signal. In an exemplary embodiment, the power point may be continuously calculated and derived from the continuous feed of dynamic reflected ultrasound signal data. In one embodiment, the power point is set a 95% power point. In another embodiment, the power point may be set at 80% or higher, 85% or higher or 90% or higher. The power point may be found by first accumulating all the values in the frequency spectrum, and then finding, from the lowest frequency to the highest, the bin where 95% of the accumulation sits. In another embodiment, the hiss noise level may be present at a certain value, and the power point may be found by first accumulating all the values in the frequency spectrum above the preset value, and then finding, from the lowest frequency to the highest, the bin where 95% of the accumulation sits. This 95% power point thus may be used to create and define a maximum frequency envelope for use in filtering thermal noise from the system. For example, For example, in one embodiment, all frequencies with magnitudes that are below the power point are filtered out. In another embodiment, alternatively or in addition to filtering out all frequencies with magnitudes that are below the power point, all frequencies below the hiss noise level are filtered out. In another embodiment, the magnitude of the frequencies that are above the power point are reduced by the hiss noise level. When SNR is relatively high (>1.5) at levels lower than this portions of the physiological blood flow signal may be confused with the noise causing loss of sound quality. However, these are extremely low level signals that do not appreciably affect clinical assessment in terms of wave shape and detail with little clinical value in terms of wave shape and detail and yet may still be heard.

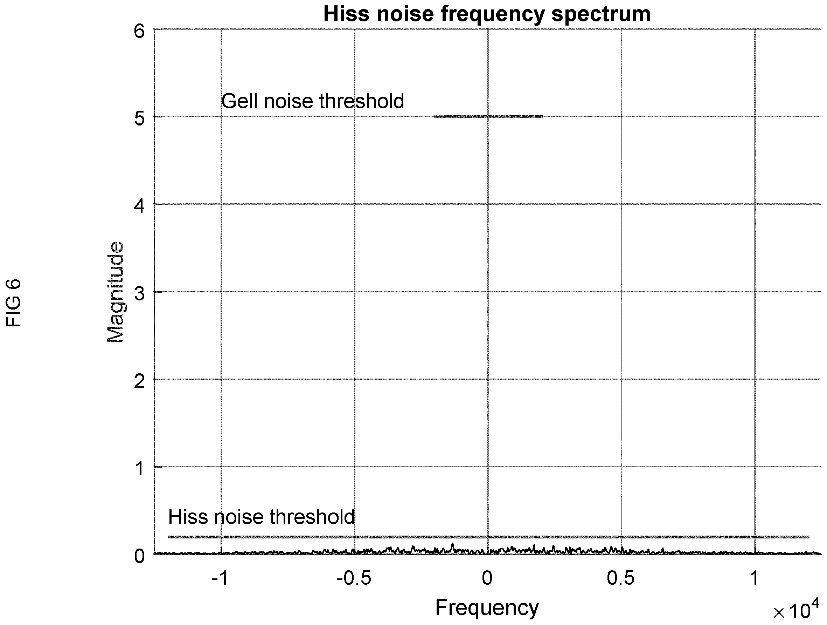

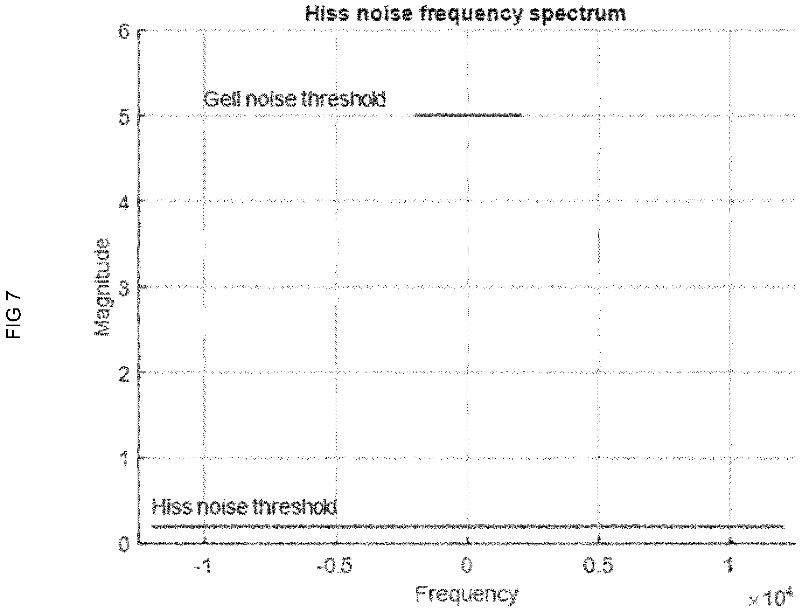

As illustrated in FIG. 4A, a thermal noise filter based on the determined maximum frequency envelope is applied to the $FFT_{complex}$ to obtain a thermal noise filtered $FFT_{complex}$ signal. FIG. 6 shows an exemplary graph of the magnitude and frequency spectrum of FIG. 5 without the thermal noise filter applied to the G1 thermal or hiss noise. The graph shows the spectrum of thermal noise from the ultrasound probe without any ultrasound gel applied. When the hiss filter is applied, the magnitude frequency spectrum is zeroed before audio reconstruction, as shown in FIG. 7. This point is used to zero all frequencies above it in the audio spectrum which, after inverse complex FFT (ICFFT), produces the audio output signal substantially without thermal noise. The thermal noise and gel filtered $FFT_{complex}$ signal is then inverted and scaled for visual waveform presentation.

The maximum frequency envelope and the $FFT_{complex}$ signal may also be used to generate a magnitude and frequency spectrogram for use in identifying the parameters in which gel suppression filter is to be activated. In stable signal acquisition, the CFFT exhibits asymmetry which changes continuously throughout the cardiac cycle with changes in flow direction and frequency content. The CFFT is performed every 10 ms on 250 samples of new IQ signal and three of the previous 10 ms samples (750 samples) in a rolling buffer of 1000 samples in total. This rolling buffer is zero padded with 24 zeros to provide the input signal for the 1024 point CFFT. The first half (512 bins) of the CFFT provides the spectrum of the forward flow components and the second half (512 bins) provides the spectral content of the reverse flow. When gel is applied to the ultrasound probe tip the CFFT becomes symmetrical, particularly in the low frequency ranges and when the SNR << 1. The symmetry and/or SNR may be exploited to provide an audio suppression value of 0 held for 1 second. After 1 second the level of the audio is linearly incremented for the next 0.5 seconds until full amplitude is achieved again (*audio suppression* = 1); unless this is interrupted by further gel noise in which case the suppression is reset to 0. The gel suppression cycle in this embodiment is thus 1.5 seconds.

To determine if gel suppression should be activated the first and last 200 values of the magnitude spectrum are averaged on an ongoing basis. If this average exceeds a predetermined threshold (e.g. 5) then the gel noise suppression cycle is initiated. In one embodiment, the predetermined gel suppression threshold value is based on dynamic continuous feed of dynamic reflected ultrasound signal data. In another embodiment, the gel suppression threshold value may be a set preset value. In yet another embodiment, the gel threshold value may be a multiple of a maximum frequency of a target physiological signal.

In one embodiment, the gel noise suppression filter may be triggered upon any one or a combination of: (a) a determination that the $FFT_{complex}$ magnitude spectrogram is substantially symmetrical; (b) when SNR is less than 1, substantially less than 1, 0.75 or less or 0.5 or less; and/or (c) when an average of the first and last set of values (e.g. first and last 200 values) of the magnitude spectrum exceeds a predetermined threshold value.

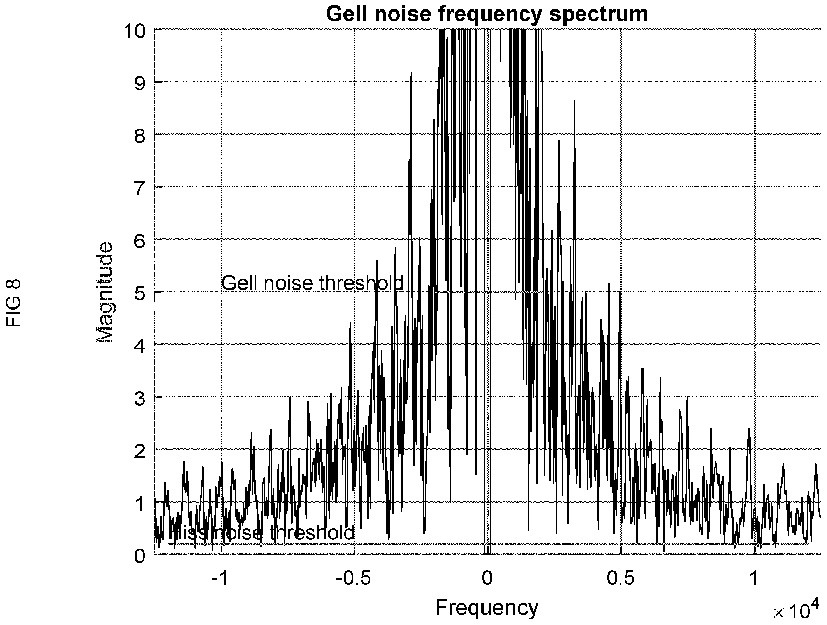

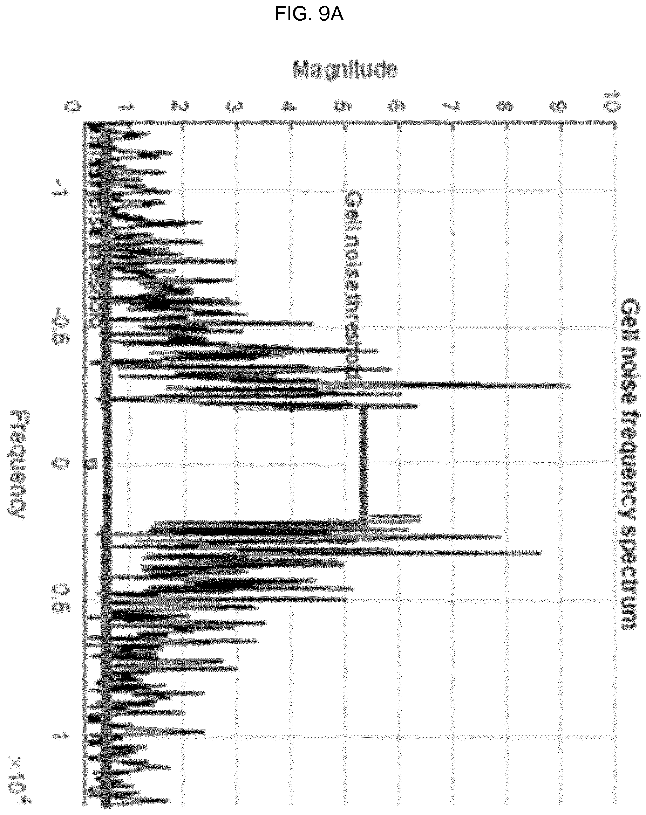

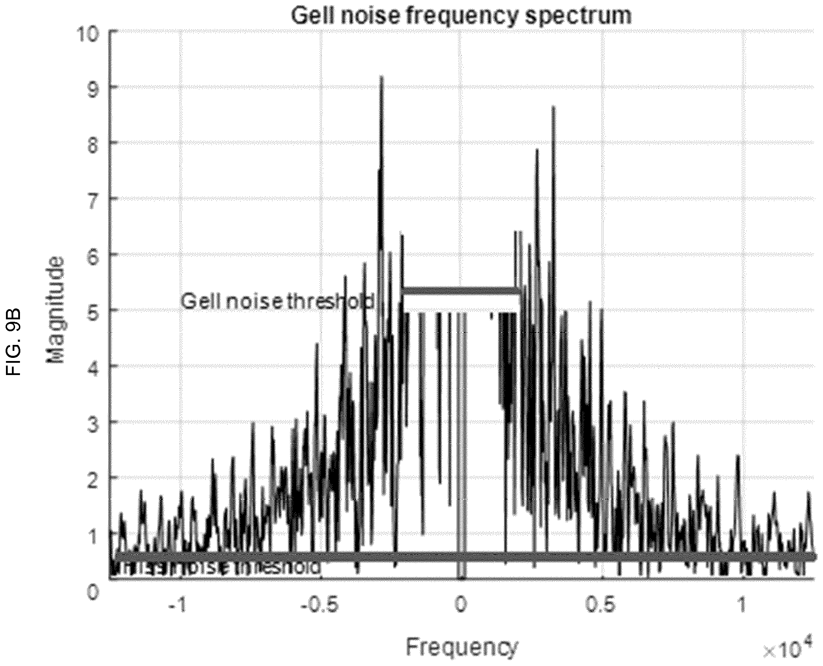

FIG. 8 shows an example of a magnitude and frequency spectrum showing a gel filter threshold value. The gel noise spectrum shows one example of substantial symmetry on either side of the zero of the frequency axis. When the gel threshold (shown in the FIG.8) is exceeded, the entire spectrum is zeroed before audio reconstruction. In another embodiment, the magnitudes are reduced by the gel threshold for the frequencies that have magnitudes that exceed the gel threshold. FIGS. 9A and 9B show an exemplary resultant signal after applying different embodiments of a gel filter.

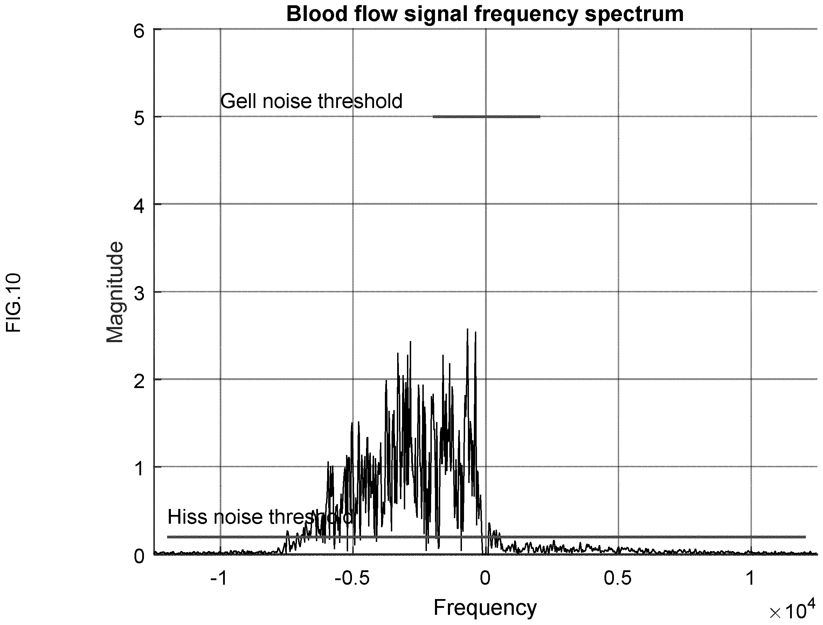

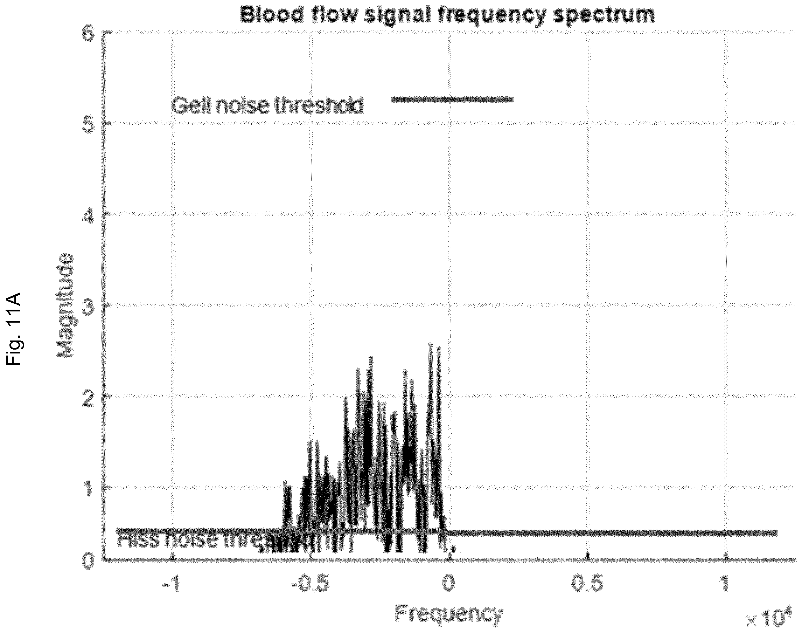

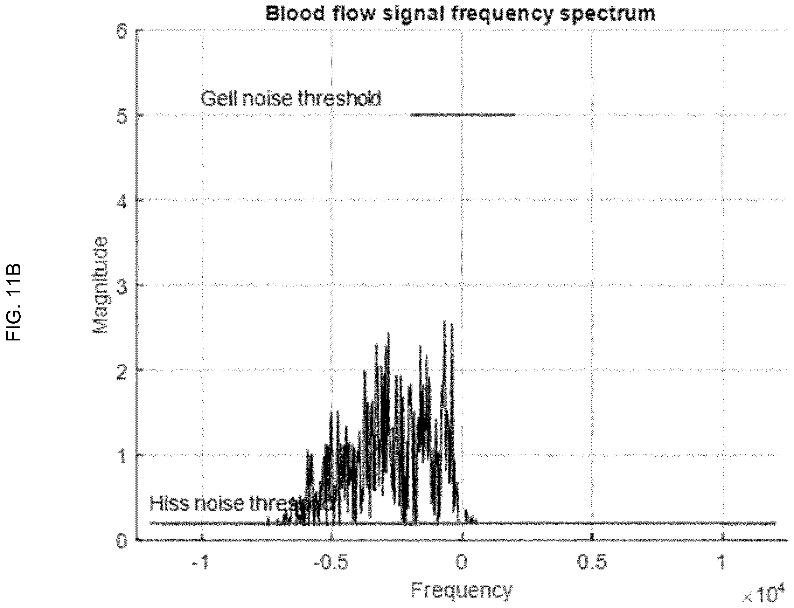

FIG. 10 shows different embodiments of a gel and thermal noise filter applied to a Doppler blood flow signal from blood detected flowing towards the Doppler probe; the graph illustrates spectral energy to the left of the graph. On the right hand side of the graph there is very little reverse flow with most of the energy lying below the hiss noise threshold. This is zeroed as shown in FIG. 11. FIG. 11 also shows that the spectrum to the left of the 95% power point is zeroed and everything to the right is preserved as a result of the $FFT_{complex}$ filter signal processing. FIGS. 11A-11B show exemplary blood flow spectrum graphs illustrating the application of different a thermal noise and gel filter to the blood flow signal.

Once the filter parameters are determined and the filter has been applied to the $FFT_{complex}$, the resultant signal may then processed in two separate ways to achieve the visual and audio resultant outputs shown in FIG. 4A. With respect to the visual output, a waveform illustrative of a property of blood flow, e.g. flow rate, may be generated. Once the maximum frequency envelope for display and possibly, a spectrogram for display is generated, from the maximum frequency envelope flow velocity parameters such as pulsatility index, blood velocity estimation etc can be calculated and/or displayed.

The second pathway for processing the $FFT_{complex}$ is configured to produce audio output. As shown in FIG. 4A, the $FFT_{complex}$ manipulates the complex spectrum into two real spectra such that when the inverse FFTs are taken will yield the forward and reverse flow audio signals. The forward and reverse signals are then passed to further analogue circuits within where they are mixed in the audio amplifier creating a mono signal to drive the integral loud speaker, or as left and right separated channels to the stereo headphones. A trained clinician can use the audio sounds to assess the quality of blood flow within a blood vessel paying attention to the power, frequencies and how these change throughout each cardiac cycle.

The blood flow waveform and corresponding audio output generated from the CFFT noise filtered signals may be used by a clinician to assess the condition of blood flow, identify stenosis and abnormalities, detect the presence of clots and aneurysms and assess therapeutic treatments available to the patient based on the visual and/or audio output of the DSP of Doppler 10.

Since the thermal and gel noise filtering process takes place in the frequency domain, thus obviating time domain convolutions and coefficient updating, a more efficient frequency band subtraction process is achieved. This gives a passband transfer function of 1 and a stopband transfer function of 0. The update time interval for the filtering process is 10 ms (i.e. every CFFT). The test for hiss noise bandwidth and gel noise are conducted for each spectrum and appropriate adjustments to each spectrum are performed.

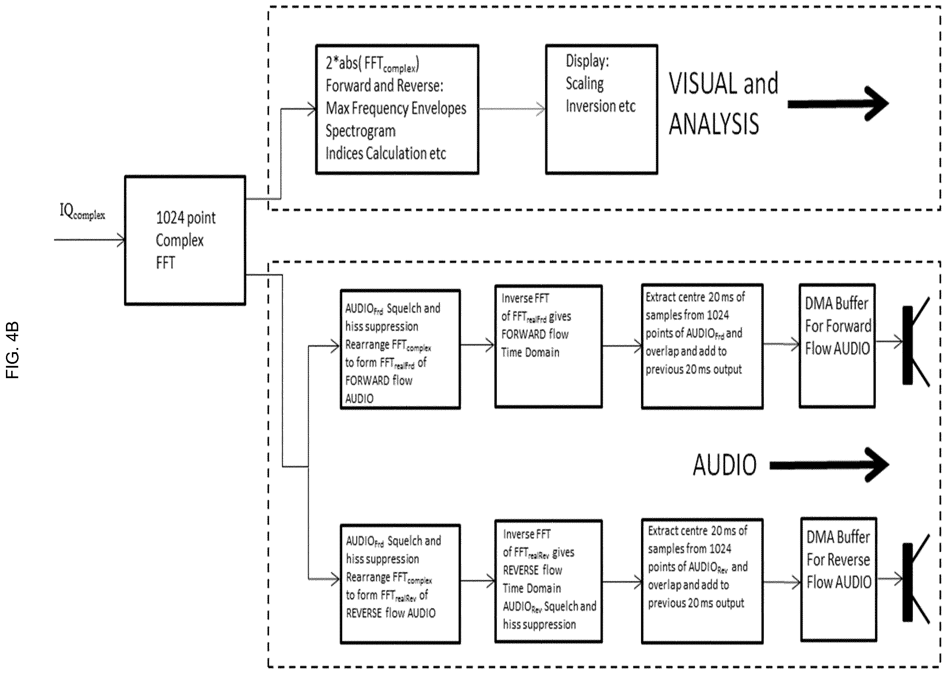

In a similar alternative embodiment shown in FIG. 4B, the maximum frequency, the filter parameters are determined while processing the $FFT_{complex}$ signals to produce a visual waveform output. These extracted parameters may be applied to the noise filters when processing the $FFT_{complex}$ signals to generate an audio output.

In an exemplary embodiment, a vascular ultrasound Doppler system may be configured to and may be used to sample I and Q signals using a Doppler probe 10, e.g. sampling at 25 ks/s; periodically calculate a complex FFT, e.g. every 10 ms; calculate forward and reverse flow maximum frequency envelopes for each complex FFT spectrum; reconstruct forward and reverse flow audio signals from the complex FFT; suppress noise arising from probe and/or gel movement; and remove thermal noise from the system.

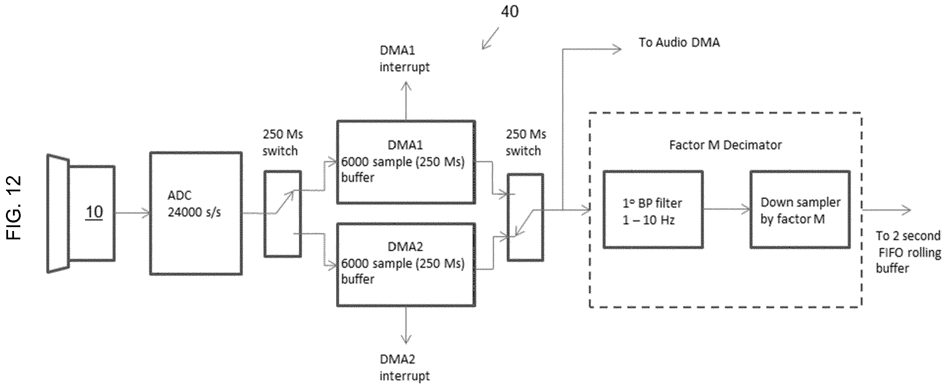

A system and methodology for filtering noise may be similarly applied using the Doppler 10 of FIG. 1 as part of an obstetric Doppler ultrasound system and digital signal processing 40 of FIGS 12-14. Unlike the vascular Doppler, the obstetric Doppler probes do not produce I and Q outputs but rather a single audio signal; directional information is not necessary for obstetric applications. This provides to the clinician a simple method for fetal heart auscultation with a rate meter in the display of the main unit. A skilled clinician can listen to umbilical artery and vein blood flow by attaching an appropriate Vascular probe. The rate meter is provided with an estimate of FHR by a method of simple low pass filtering and thresh holding. The reciprocal of the interval between each threshold crossing is used to determine the FHR.

In one embodiment, the maximum audio frequency from the obstetric probes 12 is no more than about 8 kHz. In order that FHR estimates are resolvable to within +/- 1 bpm it is necessary for the time lags of the autocorrelation function (ACF) to be resolved to 1 ms; this is particularly true at the high end of the FHR range. This involves a minimum sampling rate of 1 kHz. Therefore, the probe signal will be sampled at about 24 kHz and then decimated (ie low pass filtered and down sampled) by a factor, M, of 24.

The system uses digital signal processing (described in detail below), which allows for much of the analogue circuitry in the Doppler handheld unit to be removed. This has the dual benefit of eliminating the cost of the circuits while at the same time providing improved estimations for certain applications, e.g. fetal heart rate (FHR) determinations, and providing low cost production of separated forward and reverse blood flow audio. These cost and performance benefits are achieved by utilizing the fast Fourier transform.

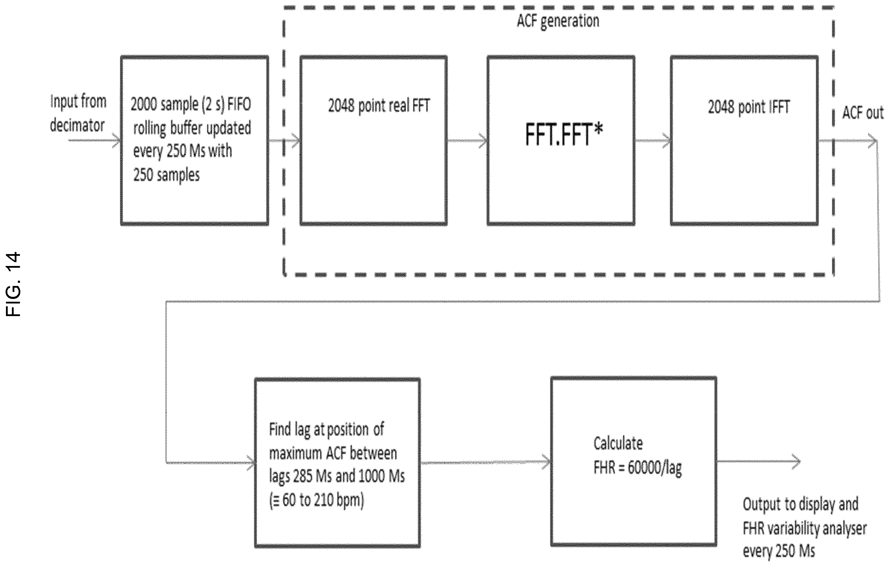

Figures 12 and 14, show an exemplary DSP flow diagram illustrating signal processing from the analogue to digital conversion through to the numerical fetal heart rate value calculating stage. There are two direct memory access (DMA) channels (DMA1 and DMA2) which alternately issue an interrupt every 250 ms when they are full with 1000 samples. The interrupt issuing DMA then passes its data to the following stages while the other DMA takes over data capture from the ADC for the next 250 ms (1000 samples). The 250 ms switches are virtual switches being implemented by the DMA controller. The 1000 samples take two paths, one unchanged to the audio output, and the other for decimation by a factor of 24 down to a sampling frequency of 1000 samples per second. The FIFO 2000 sample buffer represents a 2 second portion of signal. This buffer has to be shifted in such a way that the last 750 samples from positions 251 to 1000 are moved to over write the first 250 samples at positions 1 to 250 and the remaining 500 samples into positions 251 to 750. The positions 751 to 1000 then take the newest 250 samples. This buffer is then passed to the 2048 point FFT after padding with 48 zeros.

In the illustrated exemplary embodiment of FIG. 14, the ACF generation consists of three stages, 2048 point real FFT, point by point product of the FFT and its complex conjugate and 2048 inverse FFT. The ACF will have 2048 points which are zero symmetrical. Therefore, only the first 1024 points, representing time lags in 1 ms increments, are of interest. More specifically, lags 285 to 1000 which represent a range of FHR from 210 bpm down to 60 bpm. The appropriate lag is determined by finding the position of the maximum peak in the ACF within this range. Once the lag is found FHR is determined by taking the reciprocal of the lag multiplied by the number of milliseconds in 1 minute. The FHR value is then presented on the numeric display and could also be displayed as a FHR trace. Further FHR variability algorithms can also be applied. A FHR filter may also be applied to address any erroneous instantaneous doubling and halving of rate is not presented to the display.

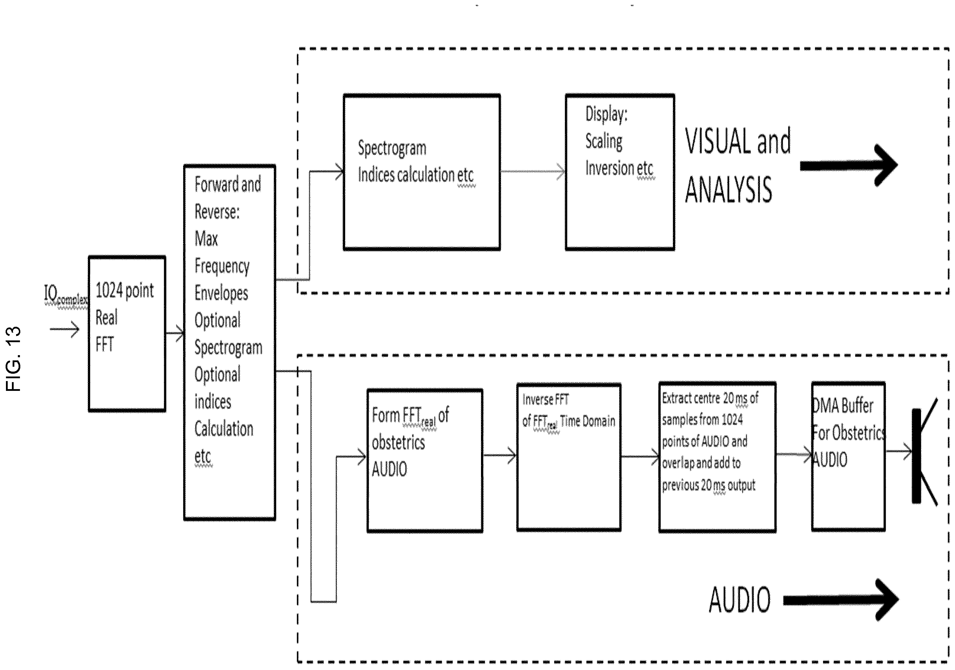

Referring to FIG 13, the similar thermal noise and gel noise filters as discussed in relation to FIGS. 2-11 may be used to improve the general accuracy and clarity of reflected fetal heart rate signals and sounds. As discussed above, the same or similar thermal noise filter derived from a maximum frequency envelope may be used to remove unwanted system and background noise. Similarly, the same or similar assessment of the magnitude and frequency spectrum of the IQ complex FFT may be conducted to determine when to apply a gel squelching filter, e.g. upon any one or a combination of:

(a) a determination that the $FFT_{real}$ magnitude spectrogram is substantially symmetrical;
(b) when SNR is less than 1, substantially less than 1, 0.75 or less or 0.5 or less; and/or
(c) when an average of the first and last set of values (e.g. first and last 200 values) of the magnitude spectrum exceeds a predetermined threshold value.

The fetal heartrate and/or vascular waveform as well as corresponding audio output generated from the FFT filtered signals of all of the above described systems may be used by a clinician to assess blood flow, condition of a blood vessel, fetal heart rate, the condition of the fetus, and assess therapeutic treatments based on the visual and/or audio output of the DSP of Doppler 10. A clinician may further diagnose a patient, identify possible therapies and/or treat a patient based on the determined blood flow and/or heartrate information.

The foregoing description has been presented for the purpose of illustration and description only and is not to be construed as limiting the scope of the disclosure in any way. The scope of the disclosure is to be determined from the claims appended hereto.

**Claims**

1. A Doppler ultrasound monitoring system (10) including a digital signal processor (20) for processing Doppler ultrasound signals reflected from a patient, wherein the signal processor (20) is configured to:

- apply a fast Fourier transform to an IQ signal derived from the reflected Doppler ultrasound signals,
- calculate a maximum frequency envelope adapted for filtering out inherent thermal system noise; and
- apply the maximum frequency envelope to the IQ signal to filter thermal noise.

2. The Doppler ultrasound monitoring system (10) of claim 1, further including a visual and/or audio system (14) for generating a visual and/or audio signal derived from the thermal noise filtered IQ signal.

3. The Doppler ultrasound monitoring system (10) of claim 2, wherein said digital signal processor is further configured to suppress an amplitude of the generated audio or visual signal for a predetermined period of time upon the occurrence of at least one of:

i) a determination that the fast Fourier transform processed IQ signal is substantially symmetrical;
ii) a dynamically assessed IQ signal to gel noise ratio is less than 1; and/or
iii) an average of a first set and last set of values of a magnitude frequency spectrum derived from the fast Fourier transform processed IQ signal exceeds a predetermined threshold indicative of gel noise.

4. The Doppler ultrasound monitoring system (10) of claim 3, wherein the digital signal processor (20) is configured to linearly increase the amplitude of the suppressed audio or visual signal until full amplitude is achieved in 0.5 seconds.

5. A method for filtering thermal noise in a handheld Doppler ultrasound system (10), including the steps of:

a) receiving Doppler ultrasound signals reflected from a patient with a transducer (12);
b) using a digital signal processor (20) to apply a fast Fourier transform to an IQ signal derived from the reflected Doppler ultrasound signals,
c) calculating a maximum frequency envelope adapted for filtering out inherent thermal system noise, and
d) applying the maximum frequency envelope to the IQ signal to filter out thermal noise.

6. The method of claim 5, further including the step of generating an audio or visual signal derived from the thermal noise filtered IQ signal.

7. The method of claim 6 further including the step of suppressing an amplitude of the generated audio and/or visual signal for a predetermined period of time upon the occurrence of at least one of:

i) a determination that the fast Fourier transform processed IQ signal is substantially symmetrical;
ii) a dynamically assessed IQ signal to gel noise ratio is less than 1; and/or
iii) an average of a first set and last set of values of a magnitude frequency spectrum derived from the fast Fourier transform processed IQ signal exceeds a predetermined threshold indicative of gel noise.

FIG. 1

FIG. 2

FIG. 3

FIG. 4A

IO_complex

1024 point Complex FFT

2*abs(FFT_complex) Forward and Reverse: Max Frequency Envelopes Optional Spectrogram Optional Indices Calculation etc

Rearrange FFT_complex to form FFT_realRev of REVERSE flow AUDIO

Rearrange FFT_complex to form FFT_realFd of FORWARD flow AUDIO

Spectrogram Indices calculation etc

Inverse FFT of FFT_realRev gives REVERSE flow Time Domain

Inverse FFT of FFT_realFd gives FORWARD flow Time Domain

Display: Scaling Inversion etc

Extract centre 20 ms of samples from 1024 points of AUDIO_Rev and overlap and add to previous 20 ms output

Extract centre 20 ms of samples from 1024 points of AUDIO_Fd and overlap and add to previous 20 ms output

DMA Buffer For Reverse Flow AUDIO

DMA Buffer For Forward Flow AUDIO

AUDIO

VISUAL and ANALYSIS

FIG. 4B

FIG. 5

Time domain signal from the I input channel

FIG 6

Hiss noise frequency spectrum

FIG 7

Hiss noise frequency spectrum

Magnitude

Gell noise threshold

Hiss noise threshold

Frequency

FIG 8

FIG. 9A

FIG. 9B

Gell noise frequency spectrum

FIG.10

Blood flow signal frequency spectrum

Magnitude

Frequency

Gell noise threshold

Hiss noise threshold

Fig. 11A

FIG. 11B

Blood flow signal frequency spectrum

Gell noise threshold

Hiss noise thresh

Magnitude

Frequency

FIG. 12

40

To Audio DMA

DMA1
interrupt

250 Ms
switch

DMA1
6000 sample (250 Ms)
buffer

250 Ms
switch

10

ADC
24000 s/s

Factor M Decimator

1° BP filter
1 – 10 Hz

Down sampler
by factor M

DMA2
6000 sample (250 Ms)
buffer

To 2 second
FIFO rolling
buffer

DMA2
interrupt

## FIG. 13

FIG. 14

Input from decimator

2000 sample (2 s) FIFO rolling buffer updated every 250 Ms with 250 samples

2048 point real FFT

FFT.FFT*

2048 point IFFT

ACF generation

ACF out

Find lag at position of maximum ACF between lags 285 Ms and 1000 Ms (≡ 60 to 210 bpm)

Calculate FHR = 60000/lag

Output to display and FHR variability analyser every 250 Ms